# EUROPEAN PATENT APPLICATION

(11) **EP 1 844 781 A1**
(43) Date of publication of application: **17.10.2007**
(21) Application number: 06384002.9
(22) Date of filing: 22.02.2006
(51) Int. Cl.: A61K 31/529, A61P 43/00

(54) **Use of sodium channel blockers for the treatment of preterm labor**

(71) Applicant: Wex Pharmaceuticals Inc., Vancouver BC V6C 1G8 (CA)
(72) Inventor: Buschmann, Helmut, H., Dr., 089060 Sant Just Desvern (ES); Noheda-Marin Pedro, 28006 Madrid (ES); Vela-Hernandez, Jose Miguel, 08041 Barcelona (ES); Candenas, Luz, 41092 Sevilla (ES)
(74) Representative: Peters, Hajo

(57) **Abstract**

The present invention refers to the use of sodium channel blockers such as tetrodotoxin or saxitoxin, its analogues/derivatives as well as their acceptable salts, for the production of a medicament for the treatment of preterm labor and/or premature birth.

## Description

### Field of the invention

The present invention refers to the use of sodium channel blockers such as tetrodotoxin or saxitoxin, its analogues/derivatives as well as their acceptable salts, for the production of a medicament for the treatment of preterm labor and/or premature birth.

### Background of the invention

Premature birth (also known as premature birth) is a big problem of our days. It is defined as birth ocurring earlier than 37 completed weeks of gestation - in comparison to 40 weeks of gestation of normal gestation. In the U.S. only 12 percent of babies are born prematurely each year - corresponding to more than 490,000 cases in the year 2003. The main reason for these premature births is the ocurrence of preterm labor; stopping premature labor is crucial for preventing preterm birth but so far no effective treatment is known.

Therefore, the objective of the present invention was to provide a new form of treatment for premature birth/preterm labor.

It has now surprisingly been found that administration of TTX is highly effective in preventing uterus contractions, which is a very promising result with respect to the treatment of premature birth and/or premature birth.

Thus, the present invention relates to the use of sodium channel blockers such as TTX or STX, its analogues/derivatives, optionally in the form of its racemate, pure stereoisomers, especially enantiomers or diastereomers or in the form of mixtures of stereoisomers, especially enantiomers or diastereomers, in any suitable mixing ratio; in neutral form, in the form of an acid or base or in form of a salt, especially a physiologically acceptable salt, or in form of a solvate, especially a hydrate for the production of a medicament for the treatment of preterm labor and/or premature birth.

The term "premature birth" as mentioned in the present invention refers to a birth occurring earlier than 37 completed weeks of gestation. Risk factors for premature birth include multiple pregnancies (twins, triplets, etc.), uterine or cervical abnormalities, certain chronic disease such as high blood pressure, kidney disease and diabetes, infections of the cervix, uterus or urinary tract, substance abuse of tobacco, alcohol and other drugs, age under 18 or over 35 years, inadequate nutrition during pregnancy, antepartum hemorrhage, pre-eclampsia or stress. As in a normal birth, in a premature birth the inner diameter of the cervix is greater than 3 cm and the length of the cervix is reduced by about 50%.

The term "preterm labor" according to the present invention refers to uterine contractions before completion of 37 weeks of gestation. Optionally, these contractions may lead to premature birth. A possible definition of preterm labor is via the frequency of uterus contractions. Preferably, preterm labor, as referred to in the present invention, is defined by a number of uterus contractions of ≥4 per hour; more preferably ≥8 contractions per hour; most preferably, the number of uterus contractions is ≥12 per hour.

The term "analogues" as used in this application is defined here as meaning a chemical compound that is a derivative of a compound which has similar biochemical activity to that compound. For example, "Analogues" of TTX bind to the same site on the alpha subunit of sodium channels as does TTX.

The term "derivatives" as used in this application is defined here as meaning a chemical compound having undergone a chemical derivation such as substitution or addition of a further chemical group to change (for pharmaceutical use) any of its physico-chemical properties, such as solubility or bioavailability. Derivatives include so-called prodrugs, e.g. ester and ether derivatives of an active compound that yield the active compound per se after administration to a subject.

Examples of well known methods of producing a prodrug of a given acting compound are known to those skilled in the art and can be found e.g. in Krogsgaard-Larsen et al., Textbook of Drugdesign and Discovery, Taylor & Francis (April 2002). "Sodium channel blockers" or "sodium channel blocking compounds" encompass any chemicals that bind selectively to a sodium channel and thereby deactivate the sodium channel. In particular they include chemicals which bind to the SS1 or SS2 extracellular domains of an alpha subunit of a sodium channel. Sodium channel blocking compounds that bind to the SS1 or SS2 subunit of a sodium channel, particularly tetrodotoxin and saxitoxin, are found to possess similar pharmaceutical activity (US 6,407,088, hereby incorporated by reference).

Tetrodotoxin (alternatively in the context of this application abbreviated TTX), also known as Ti Qu Duo Xin, is an alkaloid found in puffer fish (Tetradontiae). The chemical name is Octahydro-12-(Hydroxymethyl)-2-imino-5, 9, 7, 10a-dimethano-10aH-[1,3]dioxocino[6,5-d]pyrimidine-4,7,10,11,12-pentol with a molecular formula C₁₁H₁₇N₃O₈ and a Molecular weight of 319.27. It is a potent non-protein neurotoxin and an indispensable tool for the study of neurobiology and physiology. Tetrodotoxin (TTX) is a marine organic toxin which is mainly found in testicles, ovaries, eggs, livers, spleens, eyeballs, and blood of puffer fish as well as in diverse animal species, including goby fish, newt, frogs and the blue ringed octopus and even in marine alga. Several processes for producing TTX are known. Usually TTX is extracted from marine organisms (e.g. JP 270719 Goto and Takahashi) but besides numerous others methods of synthesis are also described (and used for the preparation of tetrodotoxin in connection to this invention) in US 6,552,191, US6,478,966, US 6,562,968 or 2002/0086997, all of which are included here by reference. Tetrodotoxin is a well known compound described for example in WO02/22129 as systemically acting as analgesic. For one of the many descriptions of TTX it is recommended turn to e.g. Tu, Anthony (Ed.) Handbook of Natural Toxins, Vol. 3: Marine Toxins and Venoms, 1988, 185-210 as well as Kao (1966), Pharmacol. Rev. 18:997 - 1049 and others.

The phrase "its (tetrodoxin's) derivatives" according to this invention is defined - using the definition of US 6,030,974 (included here by reference) - as meaning amino perhydroquinazoline compounds having the molecular formula C₁₁H₁₇N₃O₈. "Tetrodoxin's derivatives" according to this invention encompasses compounds described in US 5,846,975 (included here by reference) as amino hydrogenated quinazolines and derivatives including the substances set forth from column 3 line 40 to column 6 line 40. Specifically exemplified "derivatives of tetrodotoxin" according to this invention are including but are not limited to anhydro-tetrodotoxin, tetrodaminotoxin, methoxytetrodotoxin, ethoxytetrodotoxin, deoxytetrodotoxin and tetrodonic acid, 6 epi-tetrodotoxin, 11-deoxytetrodotoxin as well as the hemilactal type TTX derivatives (e.g. 4-*epi*-TTX, 6-*epi*-TTX, 11-*deoxy*-TTX, 4-*epi*-11-*deoxy-*TTX, TTX-8-O-hemisuccinate, chiriquitoxin, 11-*nor*-TTX-6(S)-ol, 11-*nor*-TTX-6(R)-ol, 11-*nor*-TTX-6,6-diol, 11-*oxo*-TTX and TTX-11-carboxylic acid), the lactone type TTX derivatives (e.g. 6-*epi*-TTX (lactone), 11-*deoxy*-TTX (lactone), 11-*nor*-TTX-6(S)-ol (lactone), 11-*nor*-TTX-6(R)-ol (lactone), 11-*nor*-TTX-6,6-diol (lactone), 5-*deoxy*-TTX, 5,11-*dideoxy*-TTX, 4-*epi*-5,11-*didroxy*-TTX, 1-*hydroxy*-5,11-*dideoxy*-TTX, 5,6,11-*trideoxy*-TTX and 4-*epi*-5,6,11-*trideoxy*-TTX) and the 4,9-anhydro type TTX analogs (e.g. 4,9-*anhydro*-TTX, 4,9-*anhydro*-6-*epi*-TTX, 4,9-*anhydro*-11-*deoxy*-TTX, 4,9-*anhydro*-TTX-8-*O*-hemisuccinate, 4,9-*anhydro*-TTX-11-*O*-hemisuccinate). The typical derivatives of TTX possess only 1/8 to 1/40 of the toxicity of TTX in mice, based upon bioassay in mice. It has been observed that these derivatives produce joint action, and do not interact adversely. Examples of TTX derivatives include novel TTX derivatives isolated from various organisms, as well as those that are partially or totally chemically synthesized (see e.g., Yotsu, M. et al. Agric. Biol. Chem., 53(3):893-895 (1989)).

"Derivatives and analogues of TTX", as referred to in the present invention, may include compounds having the general formula I 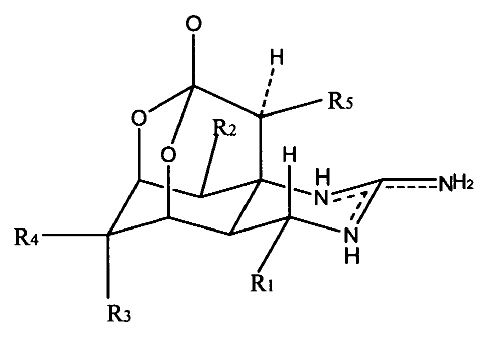 wherein, R₂ and R₅ can be selected from the group consisting of H, OH, OAC, respectively;
R₁ call be H, or an alkyl with C₁-C₄, OH, OR, OC(O)R', NH₂, NHR", NR"R"', among them R can be an alkyl with C₁-C₆, R' can be an alkyl with C₁-C₃, and R", R"' can be an alkyl with C₁-C₄, respectively;
R₃ and R₄ can be =O, or
when R₃ is H, R₄ can be selected from the group consisting of:
- ROH, and R is a branched or straight chain alkyl with C₁-C₇,
- CH(OH)NHOMe,
- NAP-gly,
- NAP-en,
- CH₂NH₂,
- CH₂NHCH₃,
- AAG,
- NMAG, and
- ANT;
when R₃ is OH or OC(O)R and R is an alkyl with C₁-C₃, R₄ can be selected from the group consisting of:
- CHO,
- CH₂-gly,
- CH₂-β-Ala,
- CH₂-Lys,
- CH₂-en,
- CH₂-NAP-Lys
- CH₂-NAP-en,
- CH(OH)CH(NH₂)COOH; and,
- NH(CH₂)ₙCOOH,
- NH(CH₂)ₙNH₂; and
- NH(CH₂)ₙCH(NH₂)COOH,
wherein:
n=1-6.
en is ethylene;
NAP is 4-triazo-2-nitrobenzoic amide, indicated as formula (a);
AAG is 2-triazo-O-aminobenzoic amide, indicated as formular (b);
NMAG is O-methylaminobenzoic amide, indicated as formula (c);
ANT is O-aminobenzoic amide, indicated as formula (d);

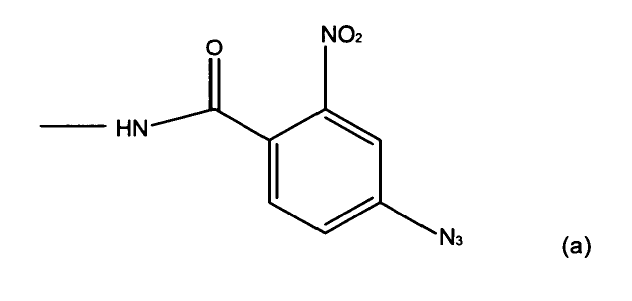

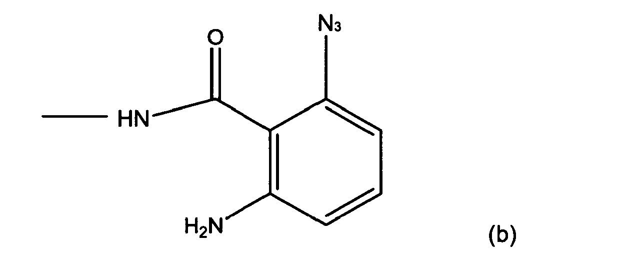

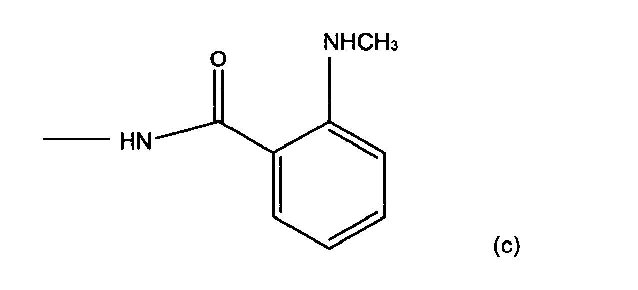

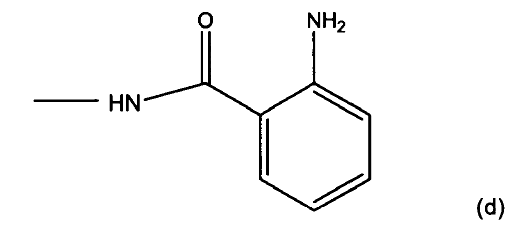

Among them, three kinds of compounds with the general formula II, III, IV are alternative.

The amino hydrogenated quinazoline compounds and derivatives thereof are compounds having following general formula II, 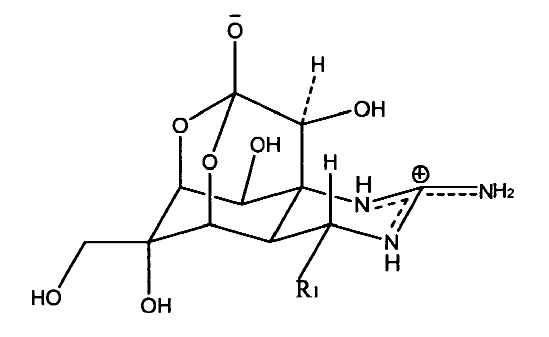 wherein: R₁ can be selected from the group consisting of OH, an alkyl or a oxyalkyl with C₁-C₄, NH₂, NHR", NR"R"', among them R" and R"' can be an alkyl with C₁ -C₄.

Among them, the more preferred compounds are:
Tetrodotoxin R₁ =OH (1);
deoxytetrodotoxin R₁=H (2);

The amino hydrogenated quiniazoline compounds and derivatives thereof are compounds having following general formula III 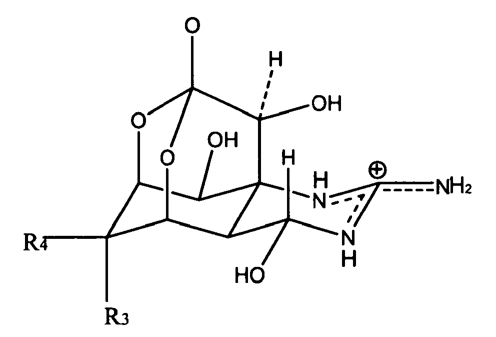 wherein:
R₃, R₄ are=O, or
when R₃ is H, R₄ is selected from the group consisting of:
CH₂OH,
CH(OH)NHOMe,
   - NAP-gly,
   - NAP-en,
   - CH₂NH₂,
   - CH₂NHCH₃,
   - AAG,
   - NMAG, and
   - ANT.

Among them, the more preferred compounds are:
AAG-degradation Tetrodotoxin R₄=AAG (3);
NMAG-degradation Tetrodotoxin R₄=NMAG (4);
ANT-degradation Tetrodotoxin R₄=ANT (5); and,
degradation Tetrodotoxin R₃, R₄ is =O (6).

The amino hydrogenated quinazoline and their derivatives are compounds having following general formula IV, 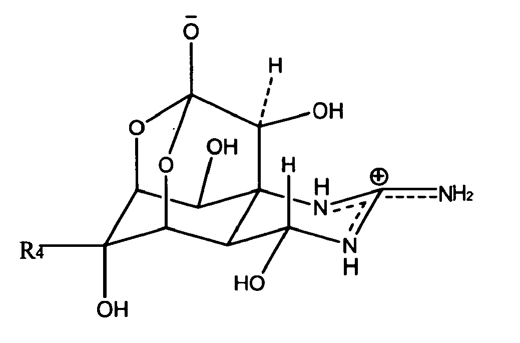 wherein, R₄ can be selected from the group consisting of:
- CHO,
- CH₂-Gly,
- CH₂-β-Ala,
- CH₂-Lys,
- CH₂-en,
- CH₂-NAP-Lys
- CH₂-NAP-en,
- -CH(OH)CH(NH₂)COOH;
- NH(CH₂)₄CH(NH₂)COOH;
- NHCH₂COOH;
- NHCH₂CH₂COOH; and
- NHCH₂CH₂NH₂.

Among them, the more preferred compounds are:
oxytetrodotoxin R₄=CHO (7);
chiriquitoxin R₄=CH(OH)CH(NH₂)COOH (8);
and the compounds with the substituted groups of R₄ :
- NH(CH₂)₄ CH(NH₂)COOH (9);
- NHCH₂COOH (10);
- NHCH₂CH₂COOH (11); and,
- NHCH₂ CH₂ NH₂ (12).

Saxitoxin (STX) and its pharmacologically acceptable salts are species of 2,6-diamino -4-((aminocarbonyl)oxy) methyl-3a,4,8,9 -tetrahydro-1H, 10H- pyrrolo(1,2-c) purine -10,10-diol (3aS-(3a-a-a-4-a, 10aR*)). The molecular formula of Saxitoxin is C₁₀H₁₇N₇O_{4,} it has a molecular weight of 299.3 and a general structure of:

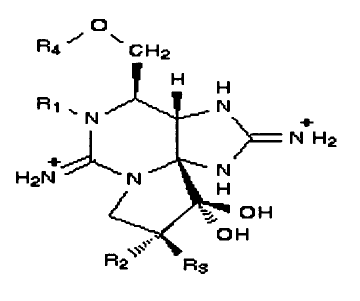

This, and its derivatives and its analogues may be used in accordance with the disclosure. Saxitoxin is readily soluble in water and can be dispersed in aerosols. It is toxic by ingestion and by inhalation, with inhalation leading to rapid respiratory collapse and death. Chemically, saxitoxin is stable, although it can be inactivated by treatment with strong alkali. It is naturally-occurring, produced by bacteria that grow in other organisms, including the dinoflagellates Gonyaulax catenella and G. tamarensis; which are consumed by the Alaskan butter clam Saxidomus giganteus and the California sea mussel, Mytilus californianeus. The toxin can be isolated from S. giganteus or M. californianeus. The first synthesis of STX was completed by Kishi and co-workers at Harvard in 1977 (J. Am. Chem. Soc. 1977, 99, 2818). A second synthesis was carried out by Jacobi and his collaborators whilst at Wesleyan University, Connecticut (J. Am. Chem. Soc. 1984, 106, 5594). A range of alternative methods for the synthesis and purification of saxitoxin will be apparent to those skilled in the art. Analogues and derivatives of saxitoxin include but are not limited to neosaxitoxin and anhydrosaxitoxin, any other biologically active variants of the above saxitoxin structure, and pharmaceutically acceptable salts thereof.

It will be appreciated that for the purposes set out herein, tetrodotoxin, saxitoxin, and their derivatives or analogues or metabolite, can be optionally in the form of their racemate, pure stereoisomers, especially enantiomers or diastereomers or in the form of mixtures of stereoisomers, especially enantiomers or diastereomers, in any suitable ratio; in neutral form, in the form of an acid or base or in form of a salt, especially a physiologically acceptable salt, or in form of a solvate, especially a hydrate.

In this application "about" means "approximately," and illustratively, the use of the term "about" indicates that dosages slightly outside the cited ranges may also be effective and safe, and such dosages are also encompassed by the scope of the present claims.

Compounds that are "administered together with TTX" or "in combination with TTX" may be administered as part of the same composition, or may be administered separately, at the same or at separate times, in the same therapeutic regimen.

In connection with this invention "neutral form" refers either to a non-ionic form or to a neutrally net charged form, for example a Zwitter-lon at its isoelectric point.

The term "salt" according to this invention is to be understood as meaning any form of the active compound according to the invention in which this compound assumes an ionic form or is charged and - if applicable - is also coupled with a counter-ion (a cation or anion). By this are also to be understood complexes of the active compound with other molecules and ions, in particular complexes which are complexed via ionic interactions. As preferred examples of salts this includes the acetate, monotrifluoracetate, acetate ester salt, citrate, formate, picrate, hydrobromide, monohydrobromide, monohydrochloride or hydrochloride.

The term "physiologically acceptable salt" in the context of this invention is understood as meaning a "salt" (as defined above) of at least one of the compounds according to the invention which are physiologically tolerated by humans and/or mammals.

The term "solvate" according to this invention is to be understood as meaning any form of the active compound according to the invention which has another molecule (most likely a polar solvent) attached to it via non-covalent bonding. Examples of solvates include hydrates and alcoholates, e.g. methanolate.

The term "treatment" or "to treat" in the context of this specification means administration of a compound or formulation according to the invention to prevent, ameliorate or eliminate one or more symptoms associated with preterm labor and/or premature birth.

Furthermore, the terms "to treat" or "treatment" according to this invention include the treatment of symptoms of preterm labor and/or premature birth, the prevention or the prophylaxis of the symptoms of preterm labor and/or premature birth, the prevention or prophylaxis causing the symptoms of preterm labor and/or premature birth, as well as the prevention or the prophylaxis of the consequences causing the symptoms.

According to the various embodiments, the sodium channel blockers such as TTX or STX, their analogues/derivatives or the pharmaceutical compositions comprising them, may be administered, in unit dosage form, intestinally, enterally, parenterally or topically, orally, subcutaneously, intranasally, by inhalation, by oral absorption, intravenously, intramuscularly, percutaneously, intraperitoneally, rectally, intravaginally, transdermally, sublingually, buccally, orally transmucosally. Administrative dosage forms may include the following: tablets, capsules, dragees, lozenges, patches, pastilles, gels, pastes, drops, aerosols, pills, powders, liquors, suspensions, emulsions, granules, ointments, creams, suppositories, freeze-dried injections, injectable compositions, in food supplements, nutritional and food bars, syrups, drinks, liquids, cordials etc, which could be regular preparation, delayed-released preparation, controlled-released preparation and various micro-granule delivery system, in food supplements, nutritional and food bars, syrups, drinks, liquids, cordials. In case of tablet, various carriers known in the art may be used, e.g. dilutent and resorbent such as starch, dextrin, calcium sulfate, kaolin, microcrystalline cellulose, aluminium silicate, etc; wetting agent and adhesives such as water, glycerin, polyethylene glycol, ethanol, propanol, starch mucilage, dextrin, syrup, honey, glucose solution, acacia, gelatin, carboxymethylcellulose sodium, shellac, methylcellulose, potassium phosphate, polyvinylpyrrolidone, etc; disintegrating agent, such as dried starch, alginate, agar powder, laminaran, sodium bicarbonate and citric acid, calcium carbonate, polyoxyethylene sorbitol aliphatic ester, lauryl sodium sulfate, methylcellulose, ethylcellulose, lactose, sucrose, maltose, mannitol, fructose, various disaccharides and polysaccharides etc; disintegration inhibiting agent, such as sucrose, tristearin, cacao butter, hydrogenated oil, etc; absorption accelerator, such as quaternary ammonium salt, lauryl sodium sulfate, etc; lubricant, such as talc, silica, corn starch, stearate, boric acid, fluid wax, polyethylene, etc. The tablet may be further formulated into coated tablet, e.g. sugar-coated tablet, film-coated tablet, enteric-coated tablet, or double-layer tablet and multi-layer tablet. In the case of pill, various carriers known in the art may be used, e.g. dilutent and resorbent, such as glucose, lactose, starch, cacao butter, hydrogenated vegetable oil, polyvinylpyrrolidone, kaolin, talc, etc; adhesives, such as acacia, bassora gum, gelatin, ethanol, honey, liquid sugar, rice paste or flour paste, etc; disintegrating agent, such as agar powder, dried starch, alginate, lauryl sodium sulfate, methylcellulose, ethylcellulose. In case of suppository, various carriers known in the art may be used, e.g. polyethylene, lecithin, cacao butter, higher alcohols, esters of higher alcohols, gelatin, semi-synthetic glyceride, etc. In the case of capsule, it may be prepared by mixing said sodium channel blockers as active ingredient with the above mentioned carriers, followed by placing the mixture into a hard gelatin capsule or soft capsule. Also, said sodium channel blockers may be applied in the following dosage forms: microcapsules, suspension in an aqueous phase, hard capsule, or injection. In the case of injection, such as liquor, emulsion, freeze-dried injection, and suspension, all the dilutents common in the art may be used, e.g. water, ethanol, polyethylene glycol, propylene glycol, oxyethylated isostearyl alcohol, polyoxidated isostearyl alcohol, polyoxyethylene sorbitol aliphatic ester, etc. In addition, in order to obtain isotonic injection, a suitable amount of sodium chloride, glucose or glycerin may be added into the preparation, as well as regular cosolvent, buffer, pH adjusting agent, etc. In addition, coloring agent, antiseptic, perfume, correctives, food sweetening agent or other materials may be added to the pharmaceutical preparation if necessary.

In alternative embodiments the sodium channel blockers such as TTX or STX, their analogues/derivatives may be administered in a schedule of one, two, three, four, five, six, seven, eight, nine, ten, eleven, twelve, thirteen, fourteen, fifteen, sixteen, seventeen, eighteen, nineteen, twenty or more doses per day, alone or in combination with other medications, over range of time periods including but not limited to periods of one, two, three, four, five, six, seven, eight, nine, ten, eleven, twelve, thirteen, fourteen, sixteen, eighteen, twenty, twenty four, thirty, or more days; or over a period of one, two, three, four, five, six, seven, eight, nine, ten, eleven, twelve, thirteen, fourteen, sixteen, eighteen, twenty, twenty four, thirty, thirty six, forty eight, sixty, seventy two, eighty four or more months.

In particular embodiments the sodium channel blockers such as TTX or STX, their analogues/derivatives may be a voltage-gated sodium channel blocker and may bind to a SS1 or SS2 α subunit of a sodium channel. The maximum daily dose of sodium channel blocker may be up to about 10µg, up to about 50µg, up to about 100µg, up to about 144µg, up to about 150µg, up to about 300µg, up to about 500µg, up to about 750µg, up to about 1000µg, up to about 1250µg, up to about 1500µg, up to about 1750µg, up to about 2000µg or more. In particular embodiments the sodium channel blocker may be administered in an amount ranging between 5 and 4000 µg/day, or in ranges between 10 and 2000µg/day, 10 and 1000µg a day, 10 and 750 µg a day, 10 and 500 µg a day, 10 and 400 µg a day, 10 and 300 µg a day, 10 and 200 µg a day, or 10 and 100 µg/day.

In particular embodiments the daily applied dose may be from about 10 to about 160µg, about 10 to about 140µg, about 10 to about 120µg, about 10 to about100 µg, _about 10 to about 90µg, about 10 to about 80µg, about 10 to about 70µg, about 10 to about 60 µg, about 10 to about 50 µg, about 10 to about 40µg, about 10 to about 30µg, or 1 to 20µg. In other embodiments the daily dosage of the sodium channel blocker may be about 0.1 to about 40µg per kilogram of body weight, about 1 to about 35µg per kilogram of body weight, about 5 to about 30µg per kilogram of body weight, about 10 to about 30µg per kilogram of body weight, about 15 to about 30µg per kilogram of body weight, about 10 to about 35µg per kilogram of body weight, or about 20 to about 40µg per kilogram of body weight. The unit dose may be within a range of about 5µg to about 2000µg and may be about 5 to about10µg, about 10 to about 15µg, about 15 to about 20µg, about 20 to about 25µg, about 25 to about 30 µg, about 30 to about 40µg, about 40µg to about 50µg, about 50µg to about 75µg, about 75 to about100µg, about 100 to about 150µg, about 150 to about 200µg, about 200 to about 250µg, about 250 to about 500µg, about 500 to about1000µg, about 1000 to about 1500µg or about 1500 to about 2000µg or more than 2000µg.

In some embodiments the effectiveness of a course of treatment of one, two, three, four, five or more doses or one, two or three days may last for up to about five, ten, fifteen, twenty, twenty five or thirty. In some embodiments dosing is only performed once every day or once every two, three, four, five, six, seven, eight, nine, ten, eleven, twelve, thirteen, fourteen, sixteen, eighteen, twenty, twenty four, thirty or more days.

According to the present disclosure, the dosage of said sodium channel blocker such as TTX or STX, their analogues/derivatives, depends on a variety of factors, including the nature and severity of the diseases, the sex, age, weight and individual reaction of the subject, the particular compound employed, the route and frequency of administration, etc. Said sodium channel blockers such as TTX or STX, their analogues/derivatives or the pharmaceutical compositions comprising them may be administered in single or divided dosage form, e.g. one to four doses per day. Those skilled in the art will readily understand and implement the changes to the treatment methods exemplified herein that are necessary or desirable to reflect varied therapeutic requirements.

A substance named as an "active ingredient" will have a purity of at least 97%. For example, a formulation said to have "500 µg of TTX as the active ingredient" may contain as much as 15 µg of anhydrotetrodotoxin as an impurity. On the other hand, a formulation said to have "500 µg of TTX and 500 µg of anhydrotetrodotoxin as active ingredients" will contain at least 485 µg of TTX and 485 µg of anhydrotetrodotoxin, but may contain as much as 30 µg of other substances as impurities of the active ingredients. Of course, substances named as other components of a formulation are not included when the purity of the active ingredient is considered.

An alternative embodiment of the present invention relates to the use of sodium channel blockers such as TTX its analogues/derivatives, optionally in the form of its racemate, pure stereoisomers, especially enantiomers or diastereomers or in the form of mixtures of stereoisomers, especially enantiomers or diastereomers, in any suitable mixing ratio; in neutral form, in the form of an acid or base or in form of a salt, especially a physiologically acceptable salt, or in form of a solvate, especially a hydrate for the production of a medicament for the treatment of preterm labor and/or premature birth.

An alternative embodiment of the present invention relates to the use of sodium channel blockers such as STX its analogues/derivatives, optionally in the form of its racemate, pure stereoisomers, especially enantiomers or diastereomers or in the form of mixtures of stereoisomers, especially enantiomers or diastereomers, in any suitable mixing ratio; in neutral form, in the form of an acid or base or in form of a salt, especially a physiologically acceptable salt, or in form of a solvate, especially a hydrate for the production of a medicament for the treatment of preterm labor and/or premature birth.

In an alternative embodiment of the invention the use according to the invention is restricted to tetrodotoxin as the active ingredient. The TTX/STX is optionally provided in the form of its racemate, pure stereoisomers, especially enantiomers or diastereomers or in the form of mixtures of stereoisomers, especially enantiomers or diastereomers, preferably in a suitable ratio; in neutral form, in the form of an acid or base or in form of a salt, especially a physiologically acceptable salt, or in form of a solvate, especially a hydrate.

In another alternative embodiment of the invention the use according to the invention is restricted to tetrodotoxin as the active ingredient, preferably in any suitable ratio in neutral form or as a salt, especially a physiologically acceptable salt.

In another alternative embodiment of the invention the use according to the invention is restricted to saxitoxin as the active ingredient, preferably in any suitable ratio in neutral form or as a salt, especially a physiologically acceptable salt.

More prefereably, tetrodotoxin is in neutral form or as a salt, especially a physiologically acceptable salt.

Preferably, the derivatives/analogues of tetrodotoxin comprise tetrodotoxin, anhydrotetrodotoxin, tetrodaminotoxin, methoxytetrodotoxin, ethoxytetrodotoxin, deoxytetrodotoxin, epi-tetrodotoxin and tetrodonic acid.

In another alternative embodiment of the invention tetrodotoxin, any of its derivatives and/or one of its analogues is used as the active ingredient in an amount between 10 µg/day and 4 mg/day.

In another alternative embodiment of the invention tetrodotoxin, its derivative or its analogue is isolated from a biological source, preferably from fish, especially puffer fish.

In another alternative embodiment of the invention tetrodotoxin, its derivative or its analogue is synthetic.

In another alternative embodiment of the invention is the use of tetrodotoxin, one of its analogues/derivatives, for the production of a medicament for the treatment of preterm labor.

In an alternative embodiment of the invention is the use of tetrodotoxin, one of its analogues/derivatives, for the production of a medicament for the treatment of premature birth.

Yet, in another alternative embodiment of the present invention tetrodotoxin reduces the number of uterus contractions to ≤12 contractions per hour, more preferably ≤8 contractions per hour, most preferably ≤4 contractions per hour.

Another alternative embodiment of the present invention refers to a method for the treatment of premature birth, said method comprising administering to a mammal TTX and/or one of its analogues or derivatives.

Another alternative embodiment of the present invention refers to a method for the treatment of preterm labor, said method comprising administering to a mammal TTX and/or one of its analogues or derivatives.

Another alternative embodiment of the present invention refers to a kit to administer TTX and/or one of its analogues or derivatives, said kit comprising said TTX and/or one of its analogues and/or derivatives channel blocker and instructions to use it to treat premature birth.

Another alternative embodiment of the present invention refers to a kit to administer TTX and/or one of its analogues or derivatives, said kit comprising said TTX and/or one of its analogues and/or derivatives channel blocker and instructions to use it to treat preterm labor.

Another alternative embodiment of the present invention refers to a kit to administer STX and/or one of its analogues or derivatives, said kit comprising said TTX and/or one of its analogues and/or derivatives channel blocker and instructions to use it to treat premature birth.

Another alternative embodiment of the present invention refers to a kit to administer STX and/or one of its analogues or derivatives, said kit comprising said TTX and/or one of its analogues and/or derivatives channel blocker and instructions to use it to treat preterm labor.

Another alternative embodiment of the present invention refers to a kit comprising TTX and/or one of its analogues or derivatives, optionally in the form of its racemate, pure stereoisomers, especially enantiomers or diastereomers or in the form of mixtures of stereoisomers, especially enantiomers or diastereomers; preferably in any suitable ratio; in neutral form, in the form of an acid or base or in form of a salt, especially a physiologically acceptable salt, or in form of a solvate, especially a hydrate.

The examples and figures in the following section describing pharmacological trials are merely illustrative and the invention cannot be considered in any way as being restricted to these applications.

### Examples

### Pharmacological Experiments

### Materials and Methods

### Animals.

Three-month-old virgin female Wistar rats purchased from Charles River Laboratories were housed with a light:dark cycle of 12h:12h and provided with food and water *ad libitum.* Animals in an undetermined stage of the ovarian cycle, or in the estrous stage of the ovarian cycle (as determined by microscopic examination of a vaginal smear), were killed humanely and the uterine horns rapidly removed, dissected free of adhering fat and mesentery and opened longitudinally.

### Organ bath studies.

Functional studies were performed essentially as described by (Pinto et al. (Endocrinology 140 2526-2532). Strips of uterine longitudinal smooth muscle similar in weight and length were prepared and mounted in tissue baths containing 4 ml of Krebs solution of the following composition (mM): NaCl 118; KCI 4.7; CaCl₂ 1.9 or 1.1; MgSO₄ 1.1; KH₂PO₄ 1.18; NaHCO₃ 25 and glucose 11. Some experiments were carried out in physiological solution with a low concentration of glucose (1 mM) or in a Ca²⁺-free medium.

### Veratridine-induced uterine contractions

Uterine strips were suspended under an initial tension of 5 mN, gassed with 95% O₂/5% CO₂ and maintained at 37°C. Mechanical responses were recorded isometrically by means of force-displacement transducers (Grass FT-03). Preparations were allowed to equilibrate for a 60 min period before addition of single or cumulative concentrations of the selective Na⁺ channel activators veratridine (1-100 µM); brevetoxin-B (0.1 nM-0.1 µM); grayanotoxin (10-60 µM) or aconitine (1-300 µM). Only one compound was tested on each tissue. At the end of the experiment, preparations were washed repeatedly and induced to contract at least two times by addition of KCl (40 mM) and metacholine (0.1 mM) at 30 min intervals. We analyzed the effect of different inhibitors on uterine contractions elicited by Naᵥ activators. Single or cumulative concentrations of an inhibitor or its vehicle (time-matched paired controls) were added to uterine strips exposed to a Naᵥ activator for different time periods. The effect of inhibitors was also assessed in uterine strips mounted in parallel and precontracted by 40 mM KCI.

### Spontaneous or electrically-induced uterine contractions

In other experiments, we studied the effect of several inhibitors on rhythmic contractions developed spontaneously by the uterine strips or induced electrically. Field electrical stimulation was delivered throµgh platinum electrodes incorporated into the tissue holders. Trains of pulses (2 ms, 25 V, 30 Hz) were applied for 5 s every 100 s, using a Grass SI 1 stimulator connected to a multiplier (Stimu-Splitter II, Med-Lab). One inhibitor (0.1 µM TTX, 1 µM atropine or 1 µM prazosin) or its solvent was assayed in time-matched paired uterine strips after 40-min suspension in the organ baths.

### Simultaneous measurement of contractile force and [Ca²⁺]ᵢ.

Small strips of myometrium (approximately 5 mm in length, 1 mm in width, 0.1 mm in thickness) were excised from the longitudinal smooth muscle layer under binocular control. The muscle strips were treated with the acetoxymethyl ester form of Fura-2 (Fura-2/AM, 20 µM, Molecular Probes, Invitrogen, Eugene, OR, USA) for 2-4 hours at room temperature. The noncytotoxic detergent pluronic acid (0.02 %, Molecular Probes) was added to increase the solubility of the Ca²⁺-sensitive fluorophore. Following the Fura-2/AM loading, the myometrial fiber was rinsed and placed in the quartz cuvette of a fluorimeter designed to measure changes in the fluorescence signals of FURA-2 simultaneously with force development in biological tissues (SLM Aminco-Bowman, Series 2, Microbeam, Barcelona, Spain) The tissue was incubated at 37°C in oxygenated (100% O₂) physiological salt solution of the following composition (mM): NaCl 154; KCI 5.6; CaCl₂ 2; MgSO₄ 1.2; glucose 11 and HEPES 11, adjusted to pH 7.4. The strip was attached to a Grass FT-03 force displacement transducer and suspended under an initial tension of 5 mN. To measure [Ca²⁺]ᵢ, the myometrial fiber was alternatively illuminated with two excitations wavelengths (340 nm and 380 nm) from a xenon lamp coupled with two monochromators. The emitted fluorescent light at the two excitation wavelengths (F340 and F380) was measured by a photomultiplier throµgh a 510-nm filter. After subtracting the background signal, obtained by adding 5 mM MnCl₂ at the end of the experiment, the fluorescence ratio (F340/F380) was used as an indicator of [Ca²⁺]ᵢ, essentially as described by Taggart et al., J. Physiol. 499, 485-496. Both contractile force and [Ca²⁺]ᵢ. were recorded by using data acquisition system and data recording software provided by the manufacturer.

All values are expressed as the mean ± SEM and, except where otherwise stated, n represents the number of rats used. Statistical analyses of data from functional studies were performed by one-way analysis of variance (ANOVA) followed by Dunnett's or Tukey's multiple comparison test and Student's unpaired t- test to compare the means of two groups (GRAPHPAD PRISM 4.0, California, USA). Statistical significance was accepted when P < 0.05.

### Results

### 1) Functional responses

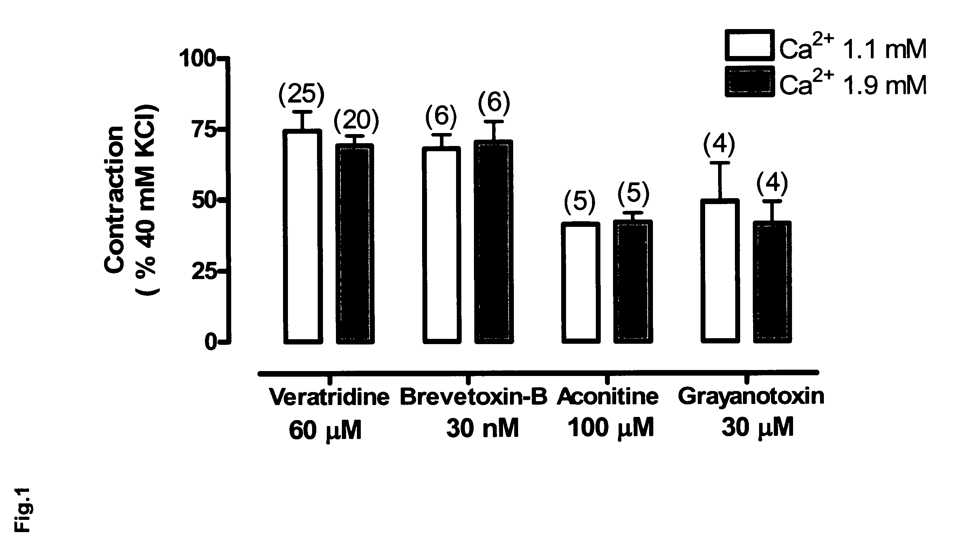

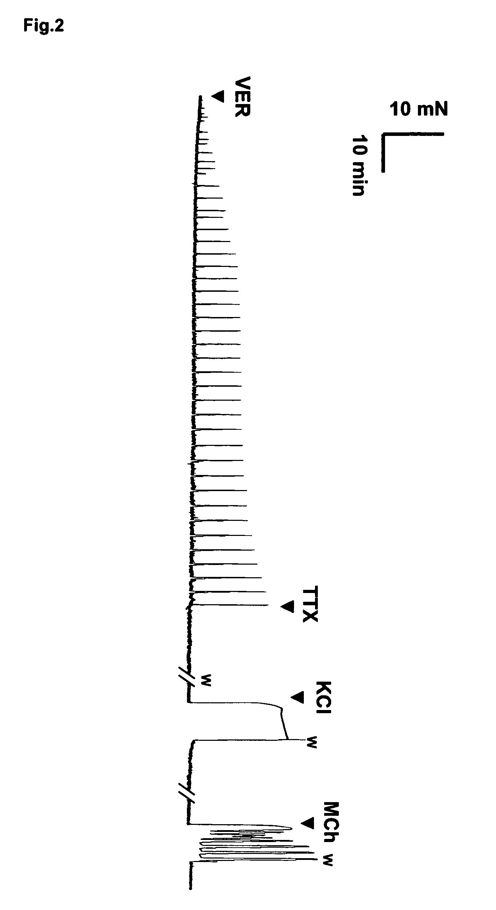

In 1.9 mM Ca²⁺-containing solution, uterine preparations obtained from non-pregnant rats in an undetermined stage of the ovarian cycle exhibited rhythmic contractions that disappeared during the first 90 min of the experiment in 80% of tissues. In a medium containing 1.1 mM Ca²⁺, the spontaneous contractions ceased during the first 90 min of the experiment in 99% of tissues. After cessation of initial activity, we analyzed the mechanical effects produced by cumulative addition of veratridine (1-100 µM), brevetoxin-B (0.1 nM-0.1 µM), grayanotoxin (10-60 µM) or aconitine (1-300 µM). The four Na⁺ channel agonists induced rhythmic contractions of irregular amplitude and frequency which appeared at concentrations ≥10 µM for veratridine (n=25), ≥10 nM for brevetoxin-B (n=6), ≥100 µM for aconitine (n=5) and ≥20 µM for grayanotoxin (n=4). The time-course and the characteristics of the contractile response elicited by each compound were similar in strips isolated from the medial, ovarian or cervical regions of the uterine horn. The maximal response, measured during a 60 min application, was reached with 60 µM veratridine, 30 nM brevetoxin, 100 µM aconitine and 30 µM grayanotoxin and was, for each compound, virtually identical in uterine strips incubated in 1.9 or 1.1 mM Ca²⁺-containing solution ***(Fig. 1).*** In all cases, addition of TTX (0.1 µM, n=75 uterine strips from 25 different rats) caused the immediate abolition of the rhythmic contractions (see ***Fig. 2).*** They were also inhibited by nifedipine (1 µM, n=12 uterine strips from 3 rats) and did not appear in uterine strips incubated in a Ca²⁺-free physiological solution containing 1 mM EGTA (n=12 uterine strips from 3 different rats). Subsequent experiments were carried out in uterine strips isolated from any region of the uterine horn and obtained from rats that were spontaneously in the estrous stage of the ovarian cycle. The physiological solution contained 1.1 mM Ca²⁺ and veratridine was used as the Naᵥ agonist.

### 2) Effects of TTX on Veratridine-induced uterine contractions

a) Addition of TTX (0.1 µM) caused the immediate abolition of the rhythmic contractions (see **Fig. 2).**
b) Additionally, we analysed the effects of the specific Naᵥ1.4 blocker µ-conotoxin GIIIB (Cruz et al., J. Biol. Chem. 260, 9280-9288), the class IC antiarrhythmic drug flecainide that blocks Naᵥ1.5 and Naᵥ1.4 (Tamargo *et al*., 1998; Desaphy *et al*., 2004) and the purported blocker of Naᵥ1.8 vinpocetine (Zhou et al., JPET, 306, 498-504). In uterine strips exposed for 90 min (late response) to 60 µM veratridine, application of cumulative concentrations of µ-conotoxin GIIIB (0.1 nM-10 µM), flecainide (1 nM-30 µM) or vinpocetine (1 nM-30 µM) did not modify the veratridine-induced rhythmic contractions (data not shown). Similar results were obtained when these blockers were applied in preparations exposed to veratridine for 40 min. Cumulative addition of TTX (0.3 nM-1 µM) caused a concentration-dependent inhibition of the early and late contraction to veratridine with -log IC₅₀ values of 8.20 ± 0.19 (n=15) and 8.02 ± 0.17 (n=11 uterine strips from 7 animals), respectively. Application of cumulative concentrations of TTX (0.3 nM-10 µM) had no effect on KCl-induced uterine contraction (data not shown).

### 3) Characterization of veratridine-induced uterine contractions.

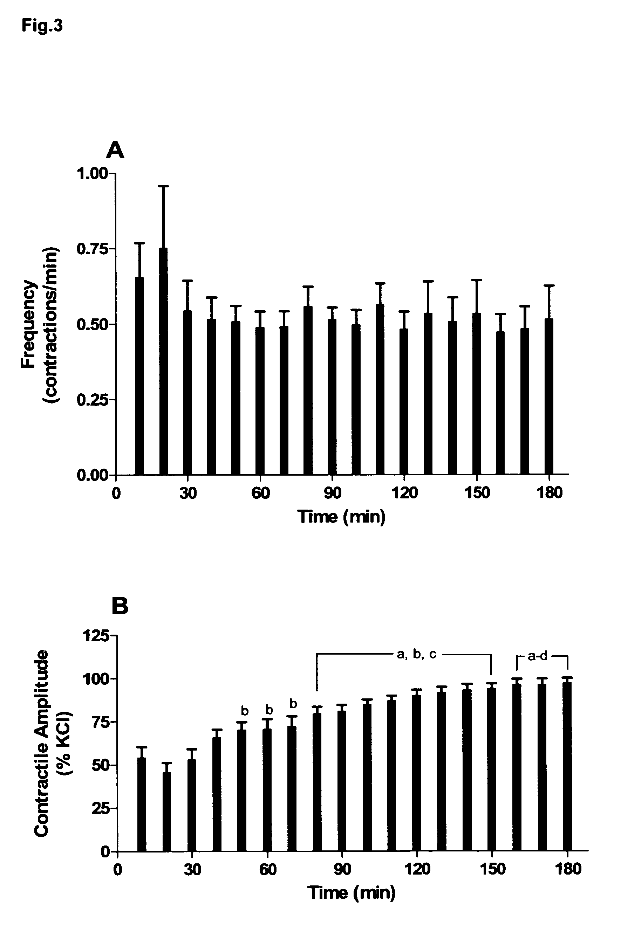

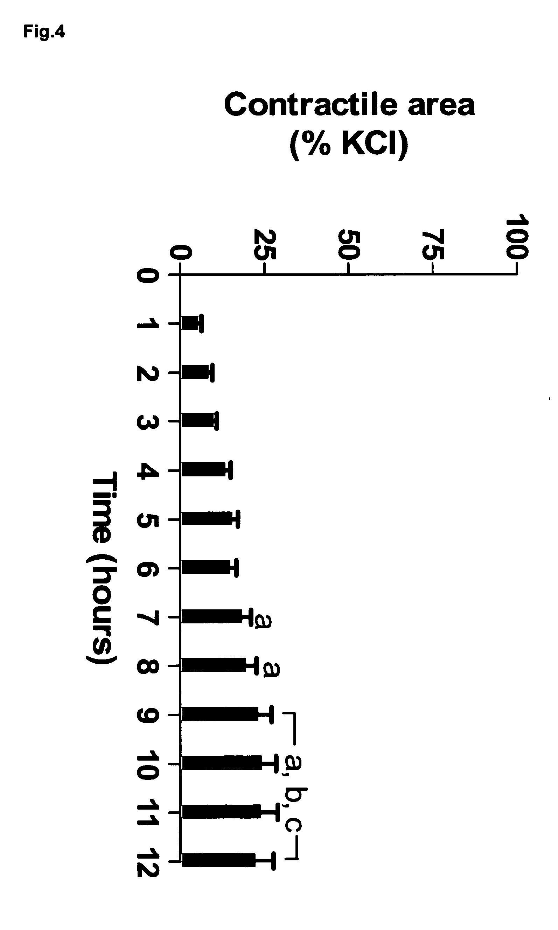

The uterine contraction to veratridine (60 µM) could be clearly divided in two different phases. The initial response was observed during the first 50-60 min of exposure and consisted of transient contractions of irregular amplitude and frequency **(Figs. 2 and 3).** In the continuous presence of veratridine, a second, late response appeared that consisted of rhythmic contractions of very regular frequency **(Figs. 2 and 3a).** The amplitude of these late contractions, and hence the area under the force-time curve, which gives a measure of the whole contractile response, slightly increased during aproximately 180 min, i.e., during the first 240 min of contact of the preparation with veratridine **(Figs. 2, 3b and 4).** From this moment, the contractile response remained very regular and well maintained for at least 12 h after addition of veratridine (24 uterine strips from 12 different animals, **Fig. 4).** The response was identical and had a similar duration time in a physiological solution containing only 1 mM glucose (*n* = 4, not shown).

### 4) Effects of TTX on spontaneous or electrically-induced uterine contractions

We also analyzed the effect of TTX (0.1 µM), atropine (1 µM) and prazosin (1 µM) on spontaneous contractions developed by the uterine strips after 40-min in physiological solution containing 1.9 mM Ca²⁺. TTX caused a decrease of the contractile area (to 66.7 ± 4.0% of the contraction during 10 min prior to the addition of TTX, n = 27) or had no effect (117.0 ± 7.2% of the previous contraction, n = 13). The TTX solvent decreased the spontaneous contraction (to 78.50 ± 2.71%, n = 20) or had no effect (128.5 ± 14.8%, n = 20). The inhibitory effect of TTX was significantly different from the inhibitory effect of the solvent (P <0.05, Student's t-test for unpaired data). Atropine caused a great inhibition (to 26.7 ± 2.2% of the previous contractile area, n = 20) while its solvent had no effect (90.3 ± 8.5%, n = 20). Atropine abolished or almost abolished spontaneous uterine contractions when added 10- or 20-min after uterine stretch. Prazosin or its solvent did not affect spontaneous activity (107.6 ± 9.1%, n = 10 and 128.7 ± 13.5%, n = 10, respectively).
TTX caused a decrease of the contractile area of electrically-induced uterine contractions (to 69.2 ± 3.0% of the contraction during 10 min prior to the addition of TTX, n = 54 uterine strips from 40 different rats). Atropine decreased the response (to 30.3 ± 8.1, n = 10) and prazosin was without effect (92.3 ± 4.0% of the previous contraction, n = 7)

### 5) Effects of TTX on contractile force and [Ca²⁺]ᵢ.

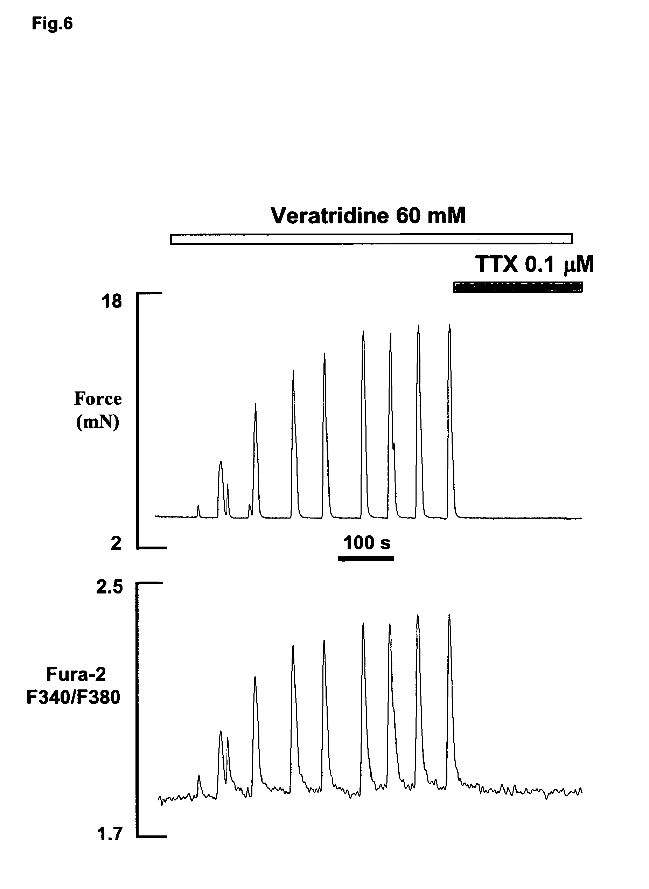

In isolated myometrial fibers loaded with Fura-2, veratridine caused rhythmic contractions accompanied by transient increases in [Ca²⁺]ᵢ. This response was observed in a half of the strips assayed (n= 10 of 21 uterine strips, obtained from 12 different rats). The mean amplitude during a 10-min period (8.48 ± 1.20 mN) and the frequency of contractions (0.75 ± 0.09 min⁻¹) were similar to values observed in uterine strips. Both the contractile force and the [Ca²⁺]ᵢ transients were abolished by addition of 0.1 µM TTX (**Fig. 6**).

### Figures

**Figure 1: Contractile responses elicited by veratridine (60 µM), brevetoxin-B (30 nM), aconitine (100 µM) and grayanotoxin (30 µM) in uterine strips from non-pregnant rats incubated in physiological solution containing 1.9 or 1.1 mM Ca²⁺.** Responses were measured as the amplitude of the rhythmic contractions during a 60 min contact period and expressed as a percentage of the maximal amplitude of the control response to KCI. Bars represent means with SEM shown by vertical lines. Numbers in parentheses indicate number of experiments in different animals.

**Figure 2: Original trace showing the typical contractile response induced by veratridine (60 µM) and its inhibition by tetrodotoxin** (TTX, 0.1 µM) in isolated uterine strips from non-pregnant rats. Reference contractions produced by KCI (40 mM) and metacholine (MCh, 0.1 mM) are also shown. w, washing.

**Figure 3: Frequency (A, contractions/min) and amplitude (B, as percentage of the KCl maximal response) of the rhythmic contractions induced by veratridine** (60 µM) in isolated uterine strips from non-pregnant rats. Responses were measured during successive intervals of 10 min, for a 180 min contact period. Bars represent means of experiments in 18 different rats, with SEM shown by vertical lines. ^{a,b,c,d}Significant differences from contractile amplitude at 10, 20, 30 and 40 min, respectively, P<0.05, one-way analysis of variance.

**Figure 4: Contractile response induced by veratridine (60 µM) in isolated uterine strips from non-pregnant rats.** Responses were measured as the area under the force-time curve during successive intervals of 1 hour, for a 12 hours contact period and were expressed as a percentage of the area to KCI during a 60 min period. Bars represent means of experiments in 24 uterine strips from 12 different rats, with SEM shown by vertical lines. ^{a,b,c}Significant differences from contractile area at 1, 2 and 3 hours, respectively, P<0.05, one-way analysis of variance.

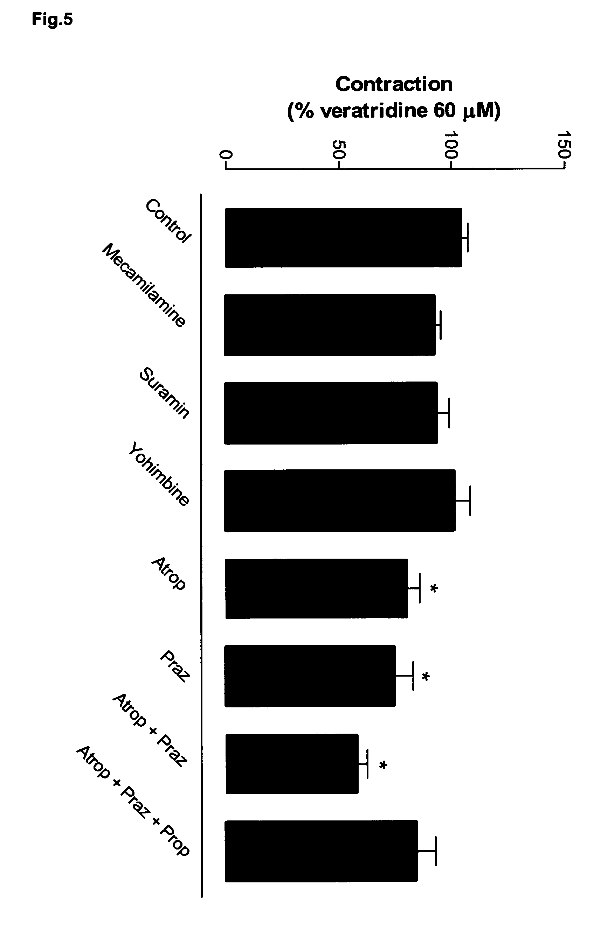

**Figure 5: Effect of mecamylamine (1 µM), suramin (100 µM), yohimbine (1 µM), atropine (Atrop, 1 µM), prazosin (Praz, 1 µM) and propanolol (Prop, 1 µM) on the veratridine-induced uterine contraction.** Responses were measured as the area under the force-time curve during a 10 min period of contact of the inhibitor or its solvent (control tissues) with the preparation and expressed as a percentage of the response to veratridine (area) during the 10 min previous to the addition of the inhibitor or solvent. Bars represent means of experiments in 5 to 8 different rats, with SEM shown by vertical lines. Significant differences from control tissues: *P< 0.05, one-way analysis of variance.

**Figure 6: Simultaneous recording of the effect of veratridine (60 µM) in contractile force** (top) and [Ca²⁺]ᵢ (Fura-2 ratio, bottom) and its inhibition by tetrodotoxin (TTX, 0.1 µM) in myometrial strips from non-pregnant rats.

### Examples of Pharmaceutical Compositions

### Orally administerable formulations

### Example 1: Capsule Formulations

| Example of a formulation (A) for a Capsule | |
|---|---|
| Tetrodotoxin (TTX) (powdered material) | 0.03 mg |
| Colloidal silicon dioxide | 0.5 mg |
| Magnesium stearate | 1.0 mg |
| Lactose | 98.47 mg |
| Total | 100 mg |
| | |

| Example of a formulation (B) for a Capsule | |
|---|---|
| Tetrodotoxin (TTX) (powdered material) | 0.15 mg |
| Colloidal silicon dioxide | 0.5 mg |
| Magnesium stearate | 1.0 mg |
| Lactose | 98.35 mg |
| Total | 100 mg |
| | |

| Example of a formulation (C) for a Capsule | |
|---|---|
| Tetrodotoxin (TTX) (powdered material) | 0.3 mg |
| Colloidal silicon dioxide | 0.5 mg |
| Magnesium stearate | 1.0 mg |
| Lactose | 98.2 mg |
| Total | 100 mg |
| | |

| Example of a formulation (D) for a Capsule | |
|---|---|
| Tetrodotoxin (TTX) (powdered material) | 0.9 mg |
| Colloidal silicon dioxide | 0.5 mg |
| Magnesium stearate | 1.0 mg |
| Lactose | 97.6 mg |
| Total | 100 mg |
| | |

| Example of a formulation (E) for a Capsule | |
|---|---|
| Tetrodotoxin (TTX) (powdered material) | 0.25 mg |
| Colloidal silicon dioxide | 0.5 mg |
| Magnesium stearate | 1.0 mg |
| Lactose | 98.25 mg |
| Total | 100 mg |
| | |

| Example of a formulation (F) for a Capsule | |
|---|---|
| Tetrodotoxin (TTX) (powdered material) | 0.5 mg |
| Colloidal silicon dioxide | 0.5 mg |
| Magnesium stearate | 1.0 mg |
| Lactose | 98.0 mg |
| Total | 100 mg |
| | |

| Example of a formulation (G) for a Capsule | |
|---|---|
| Tetrodotoxin (TTX) (powdered material) | 1.0 mg |
| Colloidal silicon dioxide | 0.5 mg |
| Magnesium stearate | 1.0 mg |
| Lactose | 97.5 mg |
| Total | 100 mg |
| | |

| Example of a formulation (H) for a Capsule | |
|---|---|
| Tetrodotoxin (TTX) (powdered material) | 1.5 mg |
| Colloidal silicon dioxide | 0.5 mg |
| Magnesium stearate | 1.0 mg |
| Lactose | 97.0 mg |
| Total | 100 mg |

### Example 2: Tablet Formulations

| Example of a formulation (A) for a tablet | |
|---|---|
| Tetrodotoxin (TTX) (powdered material) | 0.03 mg |
| Colloidal silicon dioxide | 0.5 mg |
| Magnesium stearate | 1.0 mg |
| Sodium croscarmelose | 5.0 mg |
| Lactose | 93.47 mg |
| Total | 100 mg |
| | |

| Example of a formulation (B) for a tablet | |
|---|---|
| Tetrodotoxin (TTX) (powdered material) | 0.15 mg |
| Colloidal silicon dioxide | 0.5 mg |
| Magnesium stearate | 1.0 mg |
| Sodium croscarmelose | 5.0 mg |
| Lactose | 93.35 mg |
| Total | 100 mg |
| | |

| Example of a formulation (C) for a tablet | |
|---|---|
| Tetrodotoxin (TTX) (powdered material) | 0.3 mg |
| Colloidal silicon dioxide | 0.5 mg |
| Magnesium stearate | 1.0 mg |
| Sodium croscarmelose | 5.0 mg |
| Lactose | 93.2 mg |
| Total | 100 mg |
| | |

| Example of a formulation (D) for a tablet | |
|---|---|
| Tetrodotoxin (TTX) (powdered material) | 0.9 mg |
| Colloidal silicon dioxide | 0.5 mg |
| Magnesium stearate | 1.0 mg |
| Sodium croscarmelose | 5.0 mg |
| Lactose | 92.6 mg |
| Total | 100 mg |
| | |

| Example of a formulation (E) for a tablet | |
|---|---|
| Tetrodotoxin (TTX) (powdered material) | 0.25 mg |
| Colloidal silicon dioxide | 0.5 mg |
| Magnesium stearate | 1.0 mg |
| Sodium croscarmelose | 5.0 mg |
| Lactose | 93.25 mg |
| Total | 100 mg |
| | |

| Example of a formulation (F) for a tablet | |
|---|---|
| Tetrodotoxin (TTX) (powdered material) | 0.5 mg |
| Colloidal silicon dioxide | 0.5 mg |
| Magnesium stearate | 1.0 mg |
| Sodium croscarmelose | 5.0 mg |
| Lactose | 93.0 mg |
| Total | 100 mg |
| | |

| Example of a formulation (G) for a tablet | |
|---|---|
| Tetrodotoxin (TTX) (powdered material) | 1.0 mg |
| Colloidal silicon dioxide | 0.5 mg |
| Magnesium stearate | 1.0 mg |
| Sodium croscarmelose | 5.0 mg |
| Lactose | 92.5 mg |
| Total | 100 mg |
| | |

| Example of a formulation (H) for a tablet | |
|---|---|
| Tetrodotoxin (TTX) (powdered material) | 1.5 mg |
| Colloidal silicon dioxide | 0.5 mg |
| Magnesium stearate | 1.0 mg |
| Sodium croscarmelose | 5.0 mg |
| Lactose | 92.0 mg |
| Total | 100 mg |

### Example 3: Additional Tablet Formulations

### Example of a formulation (A) for a tablet (humid Granulation)

| | |
|---|---|
| Tetrodotoxin (TTX) (powdered material) | 0.03 mg |
| Colloidal silicon dioxide | 0.5 mg |
| Magnesium stearate | 1.0 mg |
| POVIDONE K-30 | 5.0 mg |
| Sodium carboxymethylstarch | 5.0 mg |
| Microcrystalline cellulose | 20 mg |
| Lactose | 68.47 mg |
| Total | 100 mg |
| | |

| Example of a formulation (B) for a tablet (humid Granulation) | |
|---|---|
| Tetrodotoxin (TTX) (powdered material) | 0.15 mg |
| Colloidal silicon dioxide | 0.5 mg |
| Magnesium stearate | 1.0 mg |
| POVIDONE K-30 | 5.0 mg |
| Sodium carboxymethylstarch | 5.0 mg |
| Microcrystalline cellulose | 20 mg |
| Lactose | 68.35 mg |
| Total | 100 mg |
| | |

| Example of a formulation (C) for a tablet (humid Granulation) | |
|---|---|
| Tetrodotoxin (TTX) (powdered material) | 0.3 mg |
| Colloidal silicon dioxide | 0.5 mg |
| Magnesium stearate | 1.0 mg |
| POVIDONE K-30 | 5.0 mg |
| Sodium carboxymethylstarch | 5.0 mg |
| Microcrystalline cellulose | 20 mg |
| Lactose | 68.2 mg |
| Total | 100 mg |
| | |

| Example of a formulation (D) for a tablet (humid Granulation) | |
|---|---|
| Tetrodotoxin (TTX) (powdered material) | 0.9 mg |
| Colloidal silicon dioxide | 0.5 mg |
| Magnesium stearate | 1.0 mg |
| POVIDONE K-30 | 5.0 mg |
| Sodium carboxymethylstarch | 5.0 mg |
| Microcrystalline cellulose | 20 mg |
| Lactose | 67.6 mg |
| Total | 100 mg |
| | |

| Example of a formulation (E) for a tablet (humid Granulation) | |
|---|---|
| Tetrodotoxin (TTX) (powdered material) | 0.25 mg |
| Colloidal silicon dioxide | 0.5 mg |
| Magnesium stearate | 1.0 mg |
| POVIDONE K-30 | 5.0 mg |
| Sodium carboxymethylstarch | 5.0 mg |
| Microcrystalline cellulose | 20 mg |
| Lactose | 68.25 mg |
| Total | 100 mg |
| | |

| Example of a formulation (F) for a tablet (humid Granulation) | |
|---|---|
| Tetrodotoxin (TTX) (powdered material) | 0.5 mg |
| Colloidal silicon dioxide | 0.5 mg |
| Magnesium stearate | 1.0 mg |
| POVIDONE K-30 | 5.0 mg |
| Sodium carboxymethylstarch | 5.0 mg |
| Microcrystalline cellulose | 20 mg |
| Lactose | 68.0 mg |
| Total | 100 mg |
| | |

| Example of a formulation (G) for a tablet (humid Granulation) | |
|---|---|
| Tetrodotoxin (TTX) (powdered material) | 1.0 mg |
| Colloidal silicon dioxide | 0.5 mg |
| Magnesium stearate | 1.0 mg |
| POVIDONE K-30 | 5.0 mg |
| Sodium carboxymethylstarch | 5.0 mg |
| Microcrystalline cellulose | 20 mg |
| Lactose | 67.5 mg |
| Total | 100 mg |
| | |

| Example of a formulation (H) for a tablet (humid Granulation) | |
|---|---|
| Tetrodotoxin (TTX) (powdered material) | 1.5 mg |
| Colloidal silicon dioxide | 0.5 mg |
| Magnesium stearate | 1.0 mg |
| POVIDONE K-30 | 5.0 mg |
| Sodium carboxymethylstarch | 5.0 mg |
| Microcrystalline cellulose | 20 mg |
| Lactose | 67.0 mg |
| Total | 100 mg |

### Example 4: Additional Tablet Formulations

| Example of a formulation (A) for a tablet | |
|---|---|
| Tetrodotoxin (TTX) (powdered material) | 0.03 mg |
| Sodium croscarmelose (AC-DI-SOL) | 40 mg |
| Colloidal silica dioxide (AEROSYL 200) | 8 mg |
| Magnesium stearate, NF | 16 mg |
| POVIDONE K-30 | 40 mg |
| Microcrystalline cellulose (AVICEL PH-102) | 346 mg |
| Lactose monohydrate (FARMATOSE 200M) | 365.97mg |
| Total | 800 mg |
| | |

| Example of a formulation (B) for a tablet | |
|---|---|
| Tetrodotoxin (TTX) (powdered material) | 0.06 mg |
| Sodium croscarmelose (AC-DI-SOL) | 40 mg |
| Colloidal silica dioxide (AEROSYL 200) | 8 mg |
| Magnesium stearate, NF | 16 mg |
| POVIDONE K-30 | 40 mg |
| Microcrystalline cellulose (AVICEL PH-102) | 346 mg |
| Lactose monohydrate (FARMATOSE 200M) | 365.94mg |
| Total | 800 mg |
| | |

| Example of a formulation (C) for a tablet | |
|---|---|
| Tetrodotoxin (TTX) (powdered material) | 0.12 mg |
| Sodium croscarmelose (AC-DI-SOL) | 40 mg |
| Colloidal silica dioxide (AEROSYL 200) | 8 mg |
| Magnesium stearate, NF | 16 mg |
| POVIDONE K-30 | 40 mg |
| Microcrystalline cellulose (Avicel PH-102) | 346 mg |
| Lactose monohydrate (FARMATOSE 200M) | 365.88mg |
| Total | 800 mg |
| | |

| Example of a formulation (D) for a tablet | |
|---|---|
| Tetrodotoxin (TTX) (powdered material) | 0.18 mg |
| Sodium croscarmelose (AC-DI-SOL) | 40 mg |
| Colloidal silica dioxide (AEROSYL 200) | 8 mg |
| Magnesium stearate, NF | 16 mg |
| POVIDONE K-30 | 40 mg |
| Microcrystalline cellulose (Avicel PH-102) | 346 mg |
| Lactose monohydrate (FARMATOSE 200M) | 365.82mg |
| Total | 800 mg |
| | |

| Example of a formulation (E) for a tablet | |
|---|---|
| Tetrodotoxin (TTX) (powdered material) | 0.3 mg |
| Sodium croscarmelose (AC-DI-SOL) | 40 mg |
| Colloidal silica dioxide (AEROSYL 200) | 8 mg |
| Magnesium stearate, NF | 16 mg |
| POVIDONE K-30 | 40 mg |
| Microcrystalline cellulose (Avicel PH-102) | 346 mg |
| Lactose monohydrate (FARMATOSE 200M) | 365.7 mg |
| Total | 800 mg |
| | |

| Example of a formulation (F) for a tablet | |
|---|---|
| Tetrodotoxin (TTX) (powdered material) | 0.9 mg |
| Sodium croscarmelose (AC-DI-SOL) | 40 mg |
| Colloidal silica dioxide (AEROSYL 200) | 8 mg |
| Magnesium stearate, NF | 16 mg |
| POVIDONE K-30 | 40 mg |
| Microcrystalline cellulose (Avicel PH-102) | 346 mg |
| Lactose monohydrate (FARMATOSE 200M) | 365.1 mg |
| Total | 800 mg |
| | |

| Example of a formulation (G) for a tablet | |
|---|---|
| Tetrodotoxin (TTX) (powdered material) | 0.25 mg |
| Sodium croscarmelose (AC-DI-SOL) | 40 mg |
| Colloidal silica dioxide (AEROSYL 200) | 8 mg |
| Magnesium stearate, NF | 16 mg |
| POVIDONE K-30 | 40 mg |
| Microcrystalline cellulose (Avicel PH-102) | 346 mg |
| Lactose monohydrate (FARMATOSE 200M) | 365.75mg |
| Total | 800 mg |
| | |

| Example of a formulation (H) for a tablet | |
|---|---|
| Tetrodotoxin (TTX) (powdered material) | 0.5 mg |
| Sodium croscarmelose (AC-DI-SOL) | 40 mg |
| Colloidal silica dioxide (AEROSYL 200) | 8 mg |
| Magnesium stearate, NF | 16 mg |
| POVIDONE K-30 | 40 mg |
| Microcrystalline cellulose (Avicel PH-102) | 346 mg |
| Lactose monohydrate (FARMATOSE 200M) | 365.5 mg |
| Total | 800 mg |
| | |

| Example of a formulation (I) for a tablet | |
|---|---|
| Tetrodotoxin (TTX) (powdered material) | 1.0 mg |
| Sodium croscarmelose (AC-DI-SOL) | 40 mg |
| Colloidal silica dioxide (AEROSYL 200) | 8 mg |
| Magnesium stearate, NF | 16 mg |
| POVIDONE K-30 | 40 mg |
| Microcrystalline cellulose (Avicel PH-102) | 346 mg |
| Lactose monohydrate (FARMATOSE 200M) | 365.0 mg |
| Total | 800 mg |
| | |

| Example of a formulation (J) for a tablet | |
|---|---|
| Tetrodotoxin (TTX) (powdered material) | 1.5 mg |
| Sodium croscarmelose (AC-DI-SOL) | 40 mg |
| Colloidal silica dioxide (AEROSYL 200) | 8 mg |
| Magnesium stearate, NF | 16 mg |
| POVIDONE K-30 | 40 mg |
| Microcrystalline cellulose (Avicel PH-102) | 346 mg |
| Lactose monohydrate (FARMATOSE 200M) | 364.5 mg |
| Total | 800 mg |

### Example 5: Additional Tablet Formulations

| | |
|---|---|
| Example of an alternative formulation (A) for a tablet | |
| Tetrodotoxin (TTX) (powdered material) | 0.03 mg |
| Sodium croscarmelose (AC-DI-SOL) | 35 mg |
| Colloidal silica dioxide (AEROSYL 200) | 3 mg |
| Sodium stearate | 12 mg |
| Polyethylene glycol 8000 | 30 mg |
| Microcrystalline cellulose (Avicel PH-102) | 75 mg |
| Lactose monohydrate (FARMATOSE 200M) | 420.97mg |
| OPADRY II ® | 24 mg |
| Total | 600 mg |
| | |

| Example of an alternative formulation (B) for a tablet | |
|---|---|
| Tetrodotoxin (TTX) (powdered material) | 0.15 mg |
| Sodium croscarmelose (AC-DI-SOL) | 35 mg |
| Colloidal silica dioxide (AEROSYL 200) | 3 mg |
| Sodium stearate | 12 mg |
| Polyethylene glycol 8000 | 30 mg |
| Microcrystalline cellulose (Avicel PH-102) | 75 mg |
| Lactose monohydrate (FARMATOSE 200M) | 420.85mg |
| OPADRY II ® | 24 mg |
| Total | 600 mg |
| | |

| Example of an alternative formulation (C) for a tablet | |
|---|---|
| Tetrodotoxin (TTX) (powdered material) | 0.3 mg |
| Sodium croscarmelose (AC-DI-SOL) | 35 mg |
| Colloidal silica dioxide (AEROSYL 200) | 3 mg |
| Sodium stearate | 12 mg |
| Polyethylene glycol 8000 | 30 mg |
| Microcrystalline cellulose (Avicel PH-102) | 75 mg |
| Lactose monohydrate (FARMATOSE 200M) | 420.7 mg |
| OPADRY II® | 24 mg |
| Total | 600 mg |
| | |

| Example of an alternative formulation (D) for a tablet | |
|---|---|
| Tetrodotoxin (TTX) (powdered material) | 0.9 mg |
| Sodium croscarmelose (AC-DI-SOL) | 35 mg |
| Colloidal silica dioxide (AEROSYL 200) | 3 mg |
| Sodium stearate | 12 mg |
| Polyethylene glycol 8000 | 30 mg |
| Microcrystalline cellulose (Avicel PH-102) | 75 mg |
| Lactose monohydrate (FARMATOSE 200M) | 420.1 mg |
| OPADRY II ® | 24 mg |
| Total | 600 mg |
| | |

| Example of an alternative formulation (E) for a tablet | |
|---|---|
| Tetrodotoxin (TTX) (powdered material) | 0.25 mg |
| Sodium croscarmelose (AC-DI-SOL) | 35 mg |
| Colloidal silica dioxide (AEROSYL 200) | 3 mg |
| Sodium stearate | 12 mg |
| Polyethylene glycol 8000 | 30 mg |
| Microcrystalline cellulose (Avicel PH-102) | 75 mg |
| Lactose monohydrate (FARMATOSE 200M) | 420.75mg |
| OPADRY II ® | 24 mg |
| Total | 600 mg |
| | |

| Example of an alternative formulation (F) for a tablet | |
|---|---|
| Tetrodotoxin (TTX) (powdered material) | 0.5 mg |
| Sodium croscarmelose (AC-DI-SOL) | 35 mg |
| Colloidal silica dioxide (AEROSYL 200) | 3 mg |
| Sodium stearate | 12 mg |
| Polyethylene glycol 8000 | 30 mg |
| Microcrystalline cellulose (Avicel PH-102) | 75 mg |
| Lactose monohydrate (FARMATOSE 200M) | 420.5 mg |
| OPADRY II ® | 24 mg |
| Total | 600 mg |
| | |

| Example of an alternative formulation (G) for a tablet | |
|---|---|
| Tetrodotoxin (TTX) (powdered material) | 1.0 mg |
| Sodium croscarmelose (AC-DI-SOL) | 35 mg |
| Colloidal silica dioxide (AEROSYL 200) | 3 mg |
| Sodium stearate | 12 mg |
| Polyethylene glycol 8000 | 30 mg |
| Microcrystalline cellulose (Avicel PH-102) | 75 mg |
| Lactose monohydrate (FARMATOSE 200M) | 420.0 mg |
| OPADRY II ® | 24 mg |
| Total | 600 mg |
| | |

| Example of an alternative formulation (H) for a tablet | |
|---|---|
| Tetrodotoxin (TTX) (powdered material) | 1.5 mg |
| Sodium croscarmelose (AC-DI-SOL) | 35 mg |
| Colloidal silica dioxide (AEROSYL 200) | 3 mg |
| Sodium stearate | 12 mg |
| Polyethylene glycol 8000 | 30 mg |
| Microcrystalline cellulose (Avicel PH-102) | 75 mg |
| Lactose monohydrate (FARMATOSE 200M) | 419.5mg |
| OPADRY II® | 24 mg |
| Total | 600 mg |

### Example 6: Additional Capsule Formulations

| Example of an alternative formulation (A) of a capsule | |
|---|---|
| Tetrodotoxin | 0.03 mg |
| Colloidal silica dioxide | 0.8 mg |
| Magnesium stearate | 2.4 mg |
| Lactose | 476.77 mg |
| Total | 480 mg |
| | |

| Example of an alternative formulation (B) of a capsule | |
|---|---|
| Tetrodotoxin | 0.15 mg |
| Colloidal silica dioxide | 0.8 mg |
| Magnesium stearate | 2.4 mg |
| Lactose | 476.65 mg |
| Total | 480 mg |
| | |

| Example of an alternative formulation (C) of a capsule | |
|---|---|
| Tetrodotoxin | 0.3 mg |
| Colloidal silica dioxide | 0.8 mg |
| Magnesium stearate | 2.4 mg |
| Lactose | 476.5 mg |
| Total | 480 mg |
| | |

| Example of an alternative formulation (D) of a capsule | |
|---|---|
| Tetrodotoxin | 0.9 mg |
| Colloidal silica dioxide | 0.8 mg |
| Magnesium stearate | 2.4 mg |
| Lactose | 475.9 mg |
| Total | 480 mg |
| | |

| Example of an alternative formulation (E) of a capsule | |
|---|---|
| Tetrodotoxin | 0.25 mg |
| Colloidal silica dioxide | 0.8 mg |
| Magnesium stearate | 2.4 mg |
| Lactose | 476.55 mg |
| Total | 480 mg |
| | |

| Example of an alternative formulation (F) of a capsule | |
|---|---|
| Tetrodotoxin | 0.5 mg |
| Colloidal silica dioxide | 0.8 mg |
| Magnesium stearate | 2.4 mg |
| Lactose | 476.3 mg |
| Total | 480 mg |
| | |

| Example of an alternative formulation (G) of a capsule | |
|---|---|
| Tetrodotoxin | 1.0 mg |
| Colloidal silica dioxide | 0.8 mg |
| Magnesium stearate | 2.4 mg |
| Lactose | 475.8 mg |
| Total | 480 mg |
| | |

| Example of an alternative formulation (H) of a capsule | |
|---|---|
| Tetrodotoxin | 1.5 mg |
| Colloidal silica dioxide | 0.8 mg |
| Magnesium stearate | 2.4 mg |
| Lactose | 475.3 mg |
| Total | 480 mg |

### EXAMPLE 7: Outwardly Solid Formulations

### Encapsulated outwardly solid formulation (A)

| | |
|---|---|
| Tetrodotoxin | 60 mg |
| 0.5% dilute acetic acid | 1 ml |
| Acetic Acid-acetate buffer solution (pH=3.5 | 50 ml (5% of the total volume of the prepared pharmaceutical solution) |
| Water, sterile, add to | 1000 ml |

0.5 ml of this prepared solution were encapsulated in suitable consumable capsules and stored.

### Encapsulated outwardly solid formulation (B):

| | |
|---|---|
| Tetrodotoxin | 300 mg |
| 0.5% dilute acetic acid | 1 ml |
| Acetic Acid-acetate buffer solution (pH=3.5 | 50 ml (5% of the total volume of the prepared pharmaceutical solution) |
| Water, sterile, add to | 1000 ml |

0.5 ml of this prepared solution were encapsulated in suitable consumable capsules and stored.

### Encapsulated outwardly solid formulation (C)

| | |
|---|---|
| Tetrodotoxin | 600 mg |
| 0.5% dilute acetic acid | 1 ml |
| Acetic Acid-acetate buffer solution (pH=3.5 | 50 ml (5% of the total volume of the prepared pharmaceutical solution) |
| Water, sterile, add to | 1000 ml |

0.5 ml of this prepared solution were encapsulated in suitable consumable capsules and stored.

### Encapsulated outwardly solid formulation (D)

| | |
|---|---|
| Tetrodotoxin | 1800 mg |
| 0.5% dilute acetic acid | 1 ml |
| Acetic Acid-acetate buffer solution (pH=3.5 | 50 ml (5% of the total volume of the prepared pharmaceutical solution) |
| Water, sterile, add to | 1000 ml |

0.5 ml of this prepared solution were encapsulated in suitable consumable capsules and stored.

### Encapsulated outwardly solid formulation (E)

| | |
|---|---|
| Tetrodotoxin | 500 mg |
| 0.5% dilute acetic acid | 1 ml |
| Acetic Acid-acetate buffer solution (pH=3.5 | 50 ml (5% of the total volume of the prepared pharmaceutical solution) |
| Water, sterile, add to | 1000 ml |

0.5 ml of this prepared solution were encapsulated in suitable consumable capsules and stored.

### Encapsulated outwardly solid formulation (F)

| | |
|---|---|
| Tetrodotoxin | 1000 mg |
| 0.5% dilute acetic acid | 1 ml |
| Acetic Acid-acetate buffer solution (pH=3.5 | 50 ml (5% of the total volume of the prepared pharmaceutical solution) |
| Water, sterile, add to | 1000 ml |

0.5 ml of this prepared solution were encapsulated in suitable consumable capsules and stored.

### Encapsulated outwardly solid formulation (G)

| | |
|---|---|
| Tetrodotoxin | 2000 mg |
| 0.5% dilute acetic acid | 1 ml |
| Acetic Acid-acetate buffer solution (pH=3.5 | 50 ml (5% of the total volume of the prepared pharmaceutical solution) |
| Water, sterile, add to | 1000 ml |

0.5 ml of this prepared solution were encapsulated in suitable consumable capsules and stored.

### Encapsulated outwardly solid formulation (H)

| | |
|---|---|
| Tetrodotoxin | 3000 mg |
| 0.5% dilute acetic acid | 1 ml |
| Acetic Acid-acetate buffer solution (pH=3.5 | 50 ml (5% of the total volume of the prepared pharmaceutical solution) |
| Water, sterile, add to | 1000 ml |

0.5 ml of this prepared solution were encapsulated in suitable consumable capsules and stored.

### EXAMPLE 8:

### Example of a further alternative formulation of a tablet ready to be processed into an enteric-coated formulation

| | |
|---|---|
| Tetrodotoxin | 0.5 mg |
| Dibasic Calcium Phosphate USP | 46.8 mg |
| Avicel PH 101 | 50.0 mg |
| NATROSOL 250 HHX | 1.0 mg |
| CAB-O-SIL M5 | 0.5 mg |
| Magnesium Stearate NF | 1.0 mg |
| Yellow Lake F D & C No 6 | 0.2 mg |
| Purified Water USP (evaporates during the process) | |
| Total | 100 mg |

### EXAMPLE 9:

| Example of an enteric-coated version of example 8 | |
|---|---|
| Tablet according to Example 8 | 100 mg |
| Acryl-Eze yellow coating suspension House Std | 40.0 mg |

### EXAMPLE 10:

### Example of another form of tablet ready to be processed into a coated controlled-release formulation

| | |
|---|---|
| Tetrodotoxin | 0.5 mg |
| Dibasic Calcium Phosphate USP | 40.0 mg |
| Avicel PH 101 | 46.8 mg |
| NATROSOL 250 HHX | 10.0 mg |
| CAB-O-SIL M5 | 0.5 mg |
| Magnesium Stearate NF | 2.0 mg |
| Blue F D & C No1 | 0.2 mg |
| Purified Water USP (evaporates during the process) | |
| Total | 100 mg |

### EXAMPLE 11:

### Example of a coated controlled-release version of example 10

| | |
|---|---|
| Tablet according to Example 10 | 100 mg |
| SURETERIC Blue suspension House Std | 20.0 mg |
| 90/10 SURELEASE / OPADRY clear suspension | 30.0 mg |

### EXAMPLE 12:

### Example of a further alternative formulation of a tablet ready to be processed into a coated formulation

| | |
|---|---|
| Tetrodotoxin | 0.5 mg |
| Dibasic Calcium Phosphate USP | 46.0 mg |
| Avicel PH 101 | 50.0 mg |
| AC-DI-SOL | 2.0 mg |
| CAB-O-SIL M5 | 0.5 mg |
| Magnesium Stearate NF | 1.0 mg |
| Purified Water USP (evaporates during the process) | |
| Total | 100 mg |

### EXAMPLE 13:

### Example of a coated version of example 12

| | |
|---|---|
| Tablet according to Example 12 | 100 mg |
| OPADRY II coating suspension House Std | 20.0 mg |

### EXAMPLE 14:

### Example of a further alternative formulation of a tablet ready to be processed into an enteric-coated formulation

| | |
|---|---|
| Tetrodotoxin | 1.0 mg |
| Dibasic Calcium Phosphate USP | 46.3 mg |
| Avicel PH 101 | 50.0 mg |
| NATROSOL 250 HHX | 1.0 mg |
| CAB-O-SIL M5 | 0.5 mg |
| Magnesium Stearate NF | 1.0 mg |
| Yellow Lake F D & C No 6 | 0.2 mg |
| Purified Water USP (evaporates during the process) | |
| Total | 100 mg |

### EXAMPLE 15:

### Example of an enteric-coated version of example 14

| | |
|---|---|
| Tablet according to Example 14 | 100 mg |
| Acryl-Eze yellow coating suspension House Std | 40.0 mg |

### EXAMPLE 16:

### Example of another form of tablet ready to be processed into a coated controlled-release formulation

| | |
|---|---|
| Tetrodotoxin | 1.0 mg |
| Dibasic Calcium Phosphate USP | 40.0 mg |
| Avicel PH 101 | 46.3 mg |
| NATROSOL 250 HHX | 10.0 mg |
| CAB-O-SIL M5 | 0.5 mg |
| Magnesium Stearate NF | 2.0 mg |
| Blue F D & C No1 | 0.2 mg |
| Purified Water USP (evaporates during the process) | |
| Total | 100 mg |

### EXAMPLE 17:

### Example of a coated controlled-release version of example 16

| | |
|---|---|
| Tablet according to Example 16 | 100 mg |
| SURETERIC Blue suspension House Std | 20.0 mg |
| 90/10 SURELEASE / OPADRY clear suspension | 30.0 mg |

### EXAMPLE 18:

### Example of a further alternative formulation of a tablet ready to be processed into a coated formulation

| | |
|---|---|
| Tetrodotoxin | 1.0 mg |
| Dibasic Calcium Phosphate USP | 45.5 mg |
| Avicel PH 101 | 50.0 mg |
| AC-DI-SOL | 2.0 mg |
| CAB-O-SIL M5 | 0.5 mg |
| Magnesium Stearate NF | 1.0 mg |
| Purified Water USP (evaporates during the process) | |
| Total | 100 mg |

### EXAMPLE 19:

### Example of a coated version of example 18

| | |
|---|---|
| Tablet according to Example 18 | 100 mg |
| OPADRY II coating suspension House Std | 20.0 mg |

With the guidance provided herein, once the required parameters of a composition or method are known, those skilled in the art will be readily able to determine the amounts and proportions of active components and other components required to manufacture a required dosage form, manufacture a kit or composition, or use the methods and compositions disclosed. The foregoing embodiments have been described in detail by way of illustration and example for purposes of clarity and understanding. As is readily apparent to one skilled in the art, the foregoing are only some of the methods and compositions that illustrate the possible embodiments. It will be apparent to those of ordinary skill in the art that a range of equivalents, variations, changes, modifications and alterations may be applied to the compositions and methods described herein without departing from the true spirit, concept and scope of the invention.

## Claims

1. Use of tetrodotoxin, one of its analogues/derivatives, for the production of a medicament for the treatment of preterm labor and/or premature birth.

2. Use, according to claim1, **characterized in that** tetrodotoxin is optionally in the form of its racemate, pure stereoisomers, especially enantiomers or diastereomers or in the form of mixtures of stereoisomers, especially enantiomers or diastereomers, in any suitable ratio; in neutral form, in the form of an acid or base or in form of a salt, especially a physiologically acceptable salt, or in form of a solvate, especially a hydrate.

3. Use according to any of claims 1 or 2 **characterized in that** the use restricted to tetrodotoxin in neutral form or as a salt, especially a physiologically acceptable salt.

4. Use according to any of claims 1 to 3, **characterized in that** tetrodotoxin, at least one of its derivatives and/or one of its analogues is used in an amount between 10 µg/day and 4 mg/day.

5. Use according to any of claims 1 to 4, **characterized in that** tetrodotoxin, its derivatives or its analogues, are isolated from a biological source, preferably from fish, especially puffer fish.

6. Use according to any or claims 1 to 4, **characterized in that** tetrodotoxin, its derivatives or its analogues, are synthetic.

7. Use according to any of claims 1 to 6, **characterized in that** tetrodotoxin reduces the number of uterus contractions to ≤12 contractions per hour, more preferably ≤8 contractions per hour, most preferably ≤4 contractions per hour.

8. Use according to any of claims 1 to 7, **characterized in that** the use is restricted to preterm labor.

9. Use according to any of claims 1 to 7, **characterized in that** the use is restricted to premature birth.
